Europäisches Patentamt

European Patent Office

Office européen des brevets

(11) Veröffentlichungsnummer: **0 264 753 B1**

## EUROPÄISCHE PATENTSCHRIFT

(45) Veröffentlichungstag der Patentschrift: **03.02.93**

(21) Anmeldenummer: **87114820.1**

(22) Anmeldetag: **10.10.87**

(51) Int. Cl.⁵: **C12Q 1/56**, //C12Q1/37

(54) Verfahren zur Bestimmung von Plasminogen.

(30) Priorität: **16.10.86 DE 3635191**

(43) Veröffentlichungstag der Anmeldung:
**27.04.88 Patentblatt 88/17**

(45) Bekanntmachung des Hinweises auf die
Patenterteilung:
**03.02.93 Patentblatt 93/05**

(84) Benannte Vertragsstaaten:
**AT BE CH DE ES FR GB IT LI LU NL SE**

(56) Entgegenhaltungen:
**EP-A- 0 004 256**
**DE-A- 2 521 206**

**CHEMICAL ABSTRACTS, Band 82, Nr. 3, 20
Januar 1975, Columbus, OH (US); P.H.BELL
et al., Seite 169, Nr. 12776p**

**CHEMICAL ABSTRACTS, Band 99, Nr. 7, 15
August 1983, Columbus, OH (US); S.BABA et
al., Seite 186, Nr. 49324s**

**CHEMICAL ABSTRACTS, Band 92, Nr. 25, 23
Juni 1980, Columbus, OH (US); M.KNOES et
al., Seite 270, Nr. 211314t**

(73) Patentinhaber: **BEHRINGWERKE Aktiengesellschaft**
**Postfach 1140**
**W-3550 Marburg 1(DE)**

(72) Erfinder: **Kolde, Hans-Jürgen, Dr.**
**Höhenweg 54**
**W-3550 Marburg(DE)**

(74) Vertreter: **Meyer-Dulheuer, Karl-Hermann, Dr.
et al**
**HOECHST Aktiengesellschaft Postfach 3540**
**W-6200 Wiesbaden(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Bestimmung von Plasminogen mittels eines chromogenen oder fluorogenen Substrats in Gegenwart von Streptokinase.

Die Bestimmung des Plasminogens ist wegen der Bedeutung dieses Proteins für die Fibrinolyse heute ein normaler Bestandteil der Diagnose und Überwachung thrombosegefährdeter Personen. Ein Mangel an Plasminogen führt häufig zum thrombotischen Ereignis wie dem Herzinfarkt, der Lungenembolie oder tiefen Beinvenenthrombosen. Die heute häufig durchgeführte Thrombolysetherapie mit Streptokinase oder Urokinase ist ebenfalls auf eine ausreichende Konzentration von aktivierbarem Plasminogen angewiesen.

Neben immunologischen Bestimmungen mittels Nephelometrie oder radialer Immundiffusion kann Plasminogen mit fluorogenen oder chromogenen Peptidsubstraten nach Aktivierung mit Streptokinase funktionell bestimmt werden. Diese Tests nutzen es aus, daß Plasminogen nach Aktivierung zu Plasmin an Streptokinase gebunden bleibt und dieser Komplex (Aktivator) in Plasma im Gegensatz zu freiem Plasmin nicht sofort inhibiert wird. Erstmals wurde diese Technik nach Einführung chromogener Tripeptidsubstrate in Chromogenic Peptide Substrates, Chemistry and Clinical Usage, Seite 128, Scully und Kakkar ed., Churchill Livingstone, Edinburgh and New York, 1979 beschrieben. Bei dieser und weiteren ähnlichen bekannten Methoden (z.B. nach Bell et al., Anal. Biochem. 61(1), 1974, Seite 200; siehe auch Knes und Fribergen, Prog. Chem. Fibrinolysis Thrombolysis 4, 1979, Seite 154) ist jedoch eine relativ lange Vorinkubation der Probe mit Streptokinase von etwa 10 min erforderlich, um das plasmatische Plasminogen völlig in den Aktivatorkomplex zu überführen, und das Substrat wird getrennt zugegeben.

Überraschenderweise wurde gefunden, daß man Plasminogen auch in Gegenwart eines chromogenen Substrates für Plasmin durch Streptokinase aktivieren kann. Nach einer relativ kurzen Verzögerungs-Phase wird Plasmin als Komplex mit Streptokinase gebildet und das Substrat gespalten. Auch in Plasmen mit geringer Plasminogen-Konzentration setzt die Substrathydrolyse schnell ein. Der Vorteil eines solchen Verfahrens besteht darin, daß weder eine Vorinkubation noch ein zweiter Pipettierschritt zur Zugabe des Substrates erforderlich sind.

Gegenstand der Erfindung ist daher ein Verfahren zur Bestimmung von Plasminogen in einer Probe einer Körperflüssigkeit in Gegenwart von Streptokinase mittels eines Substrats für Plasmin, aus dem durch die Einwirkung des Plasmins ein optisch meßbares Spaltstück entsteht, dadurch gekennzeichnet, daß die Körperflüssigkeit ohne Vorinkubation mit Streptokinase und einem chromogenen oder fluorogenen Substrat für Plasmin versetzt und die in einer bestimmten Zeit gebildete Menge oder die Geschwindigkeit der Bildung des Spaltstücks und daraus die Konzentration von Plasminogen bestimmt wird.

Dieses Verfahren wird vorzugsweise folgendermaßen ausgeführt: Die Plasminogenkonzentration einer Probe kann mit einem Reagenz bestimmt werden, das außer einem chromogenen Substrat einen Überschuß an Streptokinase enthält, vorzugsweise 1000 E/ml. Das Plasminogen der Probe wird dabei in Plasminogen-Streptokinase-Komplexe übergeführt, die das chromogene Substrat umsetzen. Die Auswertung erfolgt bei dieser Methode durch die Bestimmung der Entstehungsgeschwindigkeit (delta E/min) des vom chromogenen Substrat abgespalteten Chromophors. Es kann aber auch die Zeit gemessen werden, die für die Entstehung einer definierten Extinktionsdifferenz, vorzugsweise 0.1 E, benötigt wird.

Bei beiden Auswertungen besteht eine Proportionalität zwischen der Plasminogenkonzentration der Probe und den gemessenen Parametern.

Ein Vergleich dieses Verfahrens (Ausführungsform 1) mit konventionellen Methoden ergab eine gute Übereinstimmung bei 40 Patientenproben (Beispiel 1).

Es kann aber auch folgendermaßen gearbeitet werden: Dazu wird ein Reagenz mit einer wesentlich geringeren Streptokinasekonzentration gebraucht. Dabei wird der Plasminogengehalt einer Probe über die Plasmin-Konzentration ermittelt, die durch die Einwirkung von Streptokinase-Plasminogen-Komplexen auf Plasminogen entsteht. Durch das Erfassen der Bildungsgeschwindigkeit von Plasmin wird neben der quantitativen Plasminogen-Bestimmung eine zusätzliche Aussage über die Aktivierbarkeit des Plasminogens möglich (Ausführungsform 2). Die Auswertung erfolgt durch die Bestimmung der Zeit, die benötigt wird, um nach Reagenzzusatz eine definierte Extinktionsdifferenz, vorzugsweise 0.1 E, zu erreichen.

An 40 Patientenplasmen konnte gezeigt werden, daß in 34 Fällen die Ergebnisse der Ausführungsformen 1 und 2 gut übereinstimmten. In 6 Fällen wurden mit der Ausführungsform 1 normale Plasminogenkonzentrationen gefunden, während die Bestimmungen mit der Ausführungsform 2 Werte unter 50 % der Norm ergaben (Beispiel 2).

Zweckmäßigerweise wird das erfindungsgemäße Verfahren in gepufferter Lösung durchgeführt, beispielsweise unter Verwendung eines Phosphat-, Tris-, Hepes- oder Acetat-Puffers mit einem pH-Wert von 6 bis 9, vorzugsweise eines Phosphatpuffers, besonders 0.1 mol/l K-Phosphat, pH 7.5.

Als chromogene Substrate sind alle chromogenen Plasminsubstrate geeignet:

H-D-CHA-NVA-Lys-pNa
H-D-Val-Leu-Lys-pNA
H-D-NVA-CHA-Lys-pNA
H-D-NLEU-CHA-Arg-pNA
H-D-But-CHA-Lys-pNA
H-D-NLEU-CHA-Lys-pNA
H-D-Phe-Tyr-Arg-pNA
Erläuterungen:

| | |
|---|---|
| CHA | Cyclohexylalanin |
| NVA | Norvalin |
| pNA | para-Nitroanilid |
| NLEU | Norleucin |
| But | epsilon-Aminobuttersäure |

Die folgenden Beispiele erläutern die Erfindung.

Beispiel 1

Streptokinase (Behringwerke AG, Marburg, Bundesrepublik Deutschland) wurde in 0,1 mol/l K-Phosphatpuffer, pH 7.5 in einer Konzentration von etwa 1000 Internat. Einheiten/ml gelöst. 4 ml dieser Lösung wurden mit 0,1 ml einer 10 mmol/l Lösung des chromogenen Substrates H-D-Cyclohexylalanyl-Norvalyl-Lysyl-para-nitroanilid (Pentapharm AG, Basel) versetzt und die Lösung auf 37°C erwärmt. 500 $\mu$l dieser Lösung wurden in einer auf 37°C vorgewärmten Küvette zu 50 $\mu$l Plasma pipettiert. Es wurde die Absorption bei 405 nm gemessen. Die Beziehung zwischen Absorption und Zeit war nach etwa 30 sec nahezu linear. Aus dem linearen Teil der Funktion wurde delta E/min bestimmt.

Herstellung von Bezugskurven:

Durch Verdünnen von Plasma mit isotoner Kochsalzlösung wurde eine Verdünnungsreihe hergestellt und jede Verdünnung wie oben beschrieben behandelt. Zur Gewinnung von Bezugskurven wurde entweder nach einer Lag-Phase von 40 sec die lineare Extinktionszunahme pro Minute oder die Zeit zum Erreichen einer bestimmten Extinktionsdifferenz benutzt.
Die Ergebnisse sind in der folgenden Tabelle dargestellt.

| Konzentration an Plasminogen (%) | delta E/min | Reaktionszeit für delta E = 0.1 in sec |
|---|---|---|
| 100 (Ausgangswert) | 0.680 | 18.0 |
| 50 | 0.360 | 31.2 |
| 25 | 0.188 | 55.8 |
| 12,5 | 0.098 | 112.8 |
| 0 | 0.000 | |

Bei Auftragung von delta E/min gegen die Konzentration wurde eine Gerade erhalten. Wenn die Zeit für eine Extinktionsdifferenz von 0.1 E halbreziprok aufgetragen wurde, wurde ebenfalls eine Gerade erhalten.

Beispiel 2

Die Durchführung der Plasminogen-Bestimmung erfolgte wie in Beispiel 1, es wurde aber ein Reagenz mit einer Streptokinase-Konzentration von 40 IE/ml eingesetzt. Mit abnehmender Streptokinase-Konzentration änderte sich die Form der Bezugskurve. Insbesondere können bei geringer Streptokinase-Konzentration, z. B. 40 IE/ml durch Messen der Reaktionszeit für ein fixes delta E von z. B. 0.1 Aussagen über die Aktivierbarkeit des Plasminogens durch Streptokinase erhalten werden. Eine solche Aussage ist für Patienten mit Myocardinfarkt von Bedeutung.

EP 0 264 753 B1

Bei vergleichenden Plasminogen-Bestimmungen in 40 Patienten-Proben nach Beispiel 1 und Beispiel 2 wurde bei insgesamt 34 Proben eine Plasminogen-Konzentration im Normalbereich gefunden, wobei die Werte beider Verfahren gut übereinstimmten. Bei 6 Proben wurden jedoch nach Beispiel 2 pathologische Werte gefunden (weniger als 50 % der Norm).

**Patentansprüche**

1. Verfahren zur Bestimmung von Plasminogen in einer Probe einer Körperflüssigkeit in Gegenwart von Streptokinase mittels eines Substrats für Plasmin, aus dem durch die Einwirkung des Plasmins ein optisch meßbares Spaltstück entsteht, dadurch gekennzeichnet, daß die Körperflüssigkeit ohne Vorinkubation mit Streptokinase und einem chromogenen oder fluorogenen Substrat für Plasmin versetzt und die in einer bestimmten Zeit gebildete Menge oder die Geschwindigkeit der Bildung des Spaltstücks und daraus die Konzentration an Plasminogen bestimmt wird.

2. Verfahren nach Anspruch 1, dadurch gekennzeichnet, daß die Körperflüssigkeit Plasma ist.

3. Verfahren nach Anspruch 1, dadurch gekennzeichnet, daß durch die Verwendung eines molaren Überschusses an Streptokinase über die vorhandene Plasminogenmenge die Gesamtkonzentration an Plasminogen bestimmt wird.

4. Verfahren nach Anspruch 1, dadurch gekennzeichnet, daß durch die Verwendung eines molaren Unterschusses an Streptokinase im Verhältnis zum vorhandenen Plasminogen die Aktivierungsgeschwindigkeit des Plasminogens bestimmt wird.

**Claims**

1. A method for the determination of plasminogen in a sample of a body fluid in the presence of streptokinase using a substrate for plasmin, the action of plasmin on which results in a cleavage product which can be measured optically, which method comprises addition of streptokinase and a chromogenic or fluorogenic substrate for plasmin to the body fluid without preincubation, and determination of the amount of the cleavage product formed in a defined time, or of the rate of formation of the cleavage product, and from this the concentration of plasminogen.

2. The method as claimed in claim 1, wherein the body fluid is plasma.

3. The method as claimed in claim 1, wherein the total concentration of plasminogen is determined by use of a molar excess of streptokinase over the amount of plasminogen present.

4. The method as claimed in claim 1, wherein the rate of activation of plasminogen is determined by use of an amount of streptokinase such that the plasminogen present is in molar excess.

**Revendications**

1. Procédé pour la détermination du plasminogène dans un échantillon d'un liquide corporel, en présence de streptokinase, au moyen d'un substrat pour la plasmine, à partir duquel se forme sous l'effet de la plasmine un fragment mesurable optiquement, caractérisé en ce que l'on ajoute au liquide corporel, sans incubation préliminaire, de la streptokinase et un substrat chromogène ou fluorogène pour la plasmine, et on détermine la quantité formée en un temps déterminé ou la vitesse de la formation du fragment, et, à partir de celle-ci, la concentration du plasminogène.

2. Procédé selon la revendication 1, caractérisé en ce que le liquide corporel est un plasma.

3. Procédé selon la revendication 1, caractérisé en ce que l'on détermine la concentration totale du plasminogène en utilisant un excès molaire de streptokinase, par rapport à la quantité présente de plasminogène.

4. Procédé selon la revendication 1, caractérisé en ce que l'on détermine la vitesse d'activation du plasminogène en utilisant la streptokinase en une quantité inférieure à la quantité molaire, par rapport

4

au plasminogène présent.